(19) Europäisches Patentamt
European Patent Office
Office européen des brevets

(11) **EP 2 714 133 B1**

(12) **EUROPÄISCHE PATENTSCHRIFT**

(45) Veröffentlichungstag und Bekanntmachung des Hinweises auf die Patenterteilung:
**05.07.2017 Patentblatt 2017/27**

(51) Int Cl.:
**A61M 1/16** (2006.01)   **A61M 1/36** (2006.01)

(21) Anmeldenummer: **12724913.4**

(22) Anmeldetag: **29.05.2012**

(86) Internationale Anmeldenummer:
**PCT/EP2012/002268**

(87) Internationale Veröffentlichungsnummer:
**WO 2012/163516 (06.12.2012 Gazette 2012/49)**

(54) **BLUTBEHANDLUNGSVORRICHTUNG**

BLOOD TREATMENT DEVICE

DISPOSITIF DE TRAITEMENT DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.05.2011 DE 102011102872**
**31.05.2011 US 201161491428 P**

(43) Veröffentlichungstag der Anmeldung:
**09.04.2014 Patentblatt 2014/15**

(73) Patentinhaber: **Fresenius Medical Care Deutschland GmbH**
**61352 Bad Homburg (DE)**

(72) Erfinder:
• **BAUER, René**
**1 Breezy Path,**
**Mid-levels,**
**Hong Kong (CN)**
• **GAGEL, Alfred**
**96123 Litzendorf (DE)**

• **KELLERMANN, Stefan**
**97711 Massbach (DE)**
• **NÜRNBERGER, Thomas**
**97705 Burkardroth (DE)**
• **SCHNEIDER, Jochen**
**97537 Wipfeld (DE)**

(74) Vertreter: **Laufhütte, Dieter et al**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
WO-A1-00/66197    DE-A1-102008 050 367
US-A- 5 437 601    US-A1- 2007 175 827
US-A1- 2007 215 545    US-A1- 2008 065 006
US-A1- 2010 268 148    US-B1- 6 610 027

EP 2 714 133 B1

**Beschreibung**

Die vorliegende Erfindung betrifft eine Blutbehandlungsvorrichtung.

Bei allen Arten der Hämodialyse wird das Blut eines an Niereninsuffizienz leidenden Patienten in einem extrakorporalen Blutkreislauf gereinigt. Das Blut wird dabei oftmals von einer okkludierenden Schlauchrollenpumpe gefördert, möglich sind aber auch andere Pumpensysteme, insbesondere Impellerpumpen (Zentrifugalpumpen).

Zumindest Teile, insbesondere die blutfördernde Pumpe, des extrakorporalen Blutkreislaufs können in ein Kassettensystem als Disposable zusammengefasst sein.

Hierbei ist die Blutflußrate ein wichtiger Parameter und kann in der Maschinensteuerung per Bedienereingabe in weiten Grenzen eingestellt werden. Hierbei sind beispielsweise Blutflußraten zwischen 30 ml/min und 600 ml/min möglich. Der richtige Blutfluß hängt beispielsweise von der Behandlungsart (Hämodialyse, Hämodiafiltration, Hämofiltration, Single-Needle-Behandlung, etc.), aber auch vom Patienten selbst ab, der individuell maximale Blutflußraten gesundheitlich toleriert.

Hierbei ist bei kindlichen Patienten besondere Vorsicht geboten, da deren Kreislauf besonders empfindlich auf zu hohe Blutflußraten des extrakorporalen Blutkreislaufs reagieren kann. Bei der pädiatrischen Behandlung darf eine bestimmte maximal infundierte Luftmenge pro Zeiteinheit nicht überschritten werden. Bei der Dialysebehandlung kann es vorkommen, daß beispielsweise durch Undichtigkeiten im Unterdruckbereich des extrakorporalen Kreislaufs Luft aus der Umgebung angesaugt wird und Luftblasen im extrakorporalen Blutkreislauf verursacht werden, deren Volumen typischerweise etwa zwischen 1 μl und 10 μl liegt. Dabei hängt das Gesamtluftvolumen pro Zeiteinheit, welches dem Patienten im Falle von Undichtigkeiten im Unterdruckbereich des extrakorporalen Blutkreislaufs infundiert wird, von der Blutflußrate ab. Je höher die Blutflußrate ist, um so höher ist im Falle von Undichtigkeiten im Unterdruckbereich des extrakorporalen Blutkreislaufs das infundierte Gesamtvolumen pro Zeiteinheit. Um die zulässige Maximalmenge an infundierter Luft pro Zeiteinheit auf keinen Fall zu überschreiten, wurde die Blutpumpenförderrate bei kindlichen Patienten bislang auf 230 ml/min begrenzt, unabhängig davon, welche körperlichen Attribute der Patient hatte.

230 ml/min ist die Blutpumpenförderrate, die dem Patienten unter 10 kg Körpergewicht gefahrlos nicht mehr als die maximal mögliche Luftmenge pro Minute zuführt. Kindliche Patienten können aber durchaus mehr als 10 kg Körpergewicht haben. Hat ein Patient beispielsweise ein Körpergewicht von 30 kg, führt die Blutpumpenratenbegrenzung auf 230 ml/min zu einer unnötig langen und deshalb für den Patienten belastenden Dialysebehandlung.

Aus der WO00/66197A1 ist es bekannt, die Ultrafiltrationsrate in Abhängigkeit von der Pulsrate und dem Blutdruck eines Patienten einzustellen. Weiterhin werden eine Mehrzahl von körperlichen Eingeschaften des Patienten überwacht.

Ein weiteres Verfahren zur Einstellung der Uktrafiltraationsrate ist aus der US2007/215545A1 bekannt.

Aus der US 2011/0017667 A1 ist bereits eine Begrenzung der Flussraten in Abhängigkeit des Körpergewichts bekannt, wobei hierbei jedoch nur auf Patienten mit einem Körpergewicht von weniger als 20 kg abgestellt wird.

Es ist daher die Aufgabe der vorliegenden Erfindung, eine Blutbehandlungsvorrichtung der eingangs genannten Art in vorteilhafter Weise weiterzubilden, insbesondere dahingehend, daß bei kindlichen Patienten eine sichere, aber zugleich wenig belastende, da mit angepasster Behandlungszeit erfolgende Behandlung ermöglicht wird.

Diese Aufgabe wird erfindungsgemäß gelöst durch eine Blutbehandlungsvorrichtung mit den Merkmalen des Anspruchs 1. Danach ist vorgesehen, daß eine Blutbehandlungsvorrichtung mit wenigstens einem Einstellmittel, mittels dessen die Blutpumpenförderrate einstellbar ist, mit einem Erfassungsmittel zur Erfassung von wenigstens einer körperlichen Eigenschaft versehen ist, wobei die körperliche Eigenschaft eine körperliche Eigenschaft eines mit der Blutbehandlungsvorrichtung zu behandelnden Patienten ist und wobei die Blutbehandlungsvorrichtung derart ausgebildet ist, daß die Art des Gefäßzugangs über eine Bedienereingabe oder eine Patientenkarte in die Blutbehandlungsvorrichtung eingebbar ist und daß mittels des Einstellmittels anhand der erfaßten körperlichen Eigenschaft und der Art des Gefäßzugangs die Blutpumpenförderrate einstellbar und/oder begrenzbar ist.

Beispielsweise kann die Blutbehandlungsmaschine eine Dialysemaschine sein.

Erfindungsgemäß ist damit vorgesehen, die Art des Gefäßzugangs über eine Bedienereingabe oder durch das Einstecken einer Patientenkarte, auf der der individuelle Gefäßzugang gespeichert ist, in die Blutbehandlungsvorrichtung einzugeben und hierdurch mit in die Einstellung und/oder Begrenzung der Blutpumpenförderrate mit eingehen zu lassen. Die Bedienereingabe betreffend den Gefäßzugang kann auch bereits darin bestehen, daß die Patienten-ID in der Blutbehandlungsvorrichtung eingegeben wird. Denkbar ist in diesem Zusammenhang weiter, daß die Daten bezüglich des Gefäßzugangs bereits in der Blutbehandlungsvorrichtung in einem Speichermittel hinterlegt sind und aufgrund der Eingabe der Patienten-ID eine diesbezügliche Zuordnung mittels bzw. seitens der Blutbehandlungsvorrichtung erfolgen kann.

In einer möglichen Ausführungsform der vorliegenden Erfindung können auch wenigstens zwei oder drei körperliche Eigenschaften erfassbar sein, anhand welcher die Blutpumpenförderrate einstellbar und/oder begrenzbar ist. Durch die Heranziehung der mindestens zwei körperlichen Eigenschaft kann auch bei kindlichen Patienten eine entsprechende Anpassung der Blutpumpenförderrate erfolgen, auch wenn diese ein Körpergewicht von mehr als 20 kg aufweisen. Im Gegensatz zu der bislang praktizierten Vorfestlegung ist nunmehr eine patientenindividuelle Einstellung der Blutpumpenförderrate möglich. Zugleich wird aber auch sichergestellt, daß insbesondere die maximal zulässige infundierte Luftmenge pro Minute nicht überschritten wird. Hierdurch wird zum Einen sichergestellt, daß der insbesondere kindliche Patient sicher behandelt werden kann, zum Anderen die Behandlung aber nicht länger als nötig erfolgen muß.

Weiterhin kann vorgesehen sein, daß eine erste und/oder eine zweite körperliche Eigenschaft die Körperoberfläche, das Körpergewicht, die Körpergröße und/oder die Blutviskosität ist. Insbesondere ist für die Höhe der Begrenzung der Blutpumpenförderrate nicht nur das Gewicht des Patienten alleine, sondern beispielsweise auch die Körperoberfläche des Patienten relevant. Eine Berechnung der Köperoberfläche kann beispielsweise gemäß nachstehend erläuterten Berechnungsmethoden erfolgen. Mittels der vorteilhafterweise integrierten Mittel zur Erfassung der körperlichen Eigenschaften, wie beispielsweise der Körpergröße und des Körpergewichts ist die Blutbehandlungsvorrichtung dazu eingerichtet, mit Hilfe dieser gemessenen Daten die Körperoberfläche beispielsweise zu bestimmen und entsprechend die Förderrate der Blutpumpe automatisch zu begrenzen. Derart ausgestaltet muß der Bediener vorteilhafterweise keine Körperdaten eingeben, was die Bequemlichkeit erhöht und die Gefahr von Fehleingaben verhindert. Weiterhin kann die Blutpumpenförderrate auch durch andere Parameter begrenzt werden. Insbesondere kann die Blutviskosität herangezogen werden. Die Blutviskosität steht zwar primär nicht in direktem Zusammenhang mit der infundierten Luftmenge, je dicker das Patientenblut aber ist, um so weniger gut strömt es im extrakorporalen Blutkreislauf. Ebenfalls können die Komponenten des extrakorporalen Blutkreislaufs begrenzend auf die Förderrate der Blutpumpe wirken. Beispielsweise ermöglicht ein einfacher Katheterzugang nur kleinere Blutförderraten als ein Fistelzugang oder der Zugang über ein künstliches Gefäßstück.

Insbesondere kann vorgesehen sein, daß die körperliche Eigenschaft die Körperoberfläche ist. Aus der Information und Kombination der beiden Eigenschaften Körperoberfläche und Gefäßzugang läßt sich besonders vorteilhaft die Blutpumpenförderrate zweckmäßig einstellen, d. h. unter Sicherheitsgesichtspunkten auf der einen und hinsichtlich der bestmöglichen Behandlungsdauer auf der anderen Seite bestimmen und begrenzen. Durch die Art des Gefäßzugangs könnten der Einfluss dieser Eigenschaft bei der Bestimmung der Blutpumpenförderrate berücksichtigt werden und die Berücksichtigung der Körperoberfläche des Patienten ermöglicht es, die zulässige Maximalmenge an infundierter Luft pro Zeiteinheit auf keinen Fall zu überschreiten.

Des Weiteren kann vorgesehen sein, daß die erste, zweite und eine dritte körperliche Eigenschaft Körperoberfläche, Körpergewicht und Größe oder dass eine erste und eine zweite körperliche Eigenschaft Körpergewicht und Größe sind oder daß eine erste, zweite und eine dritte körperliche Eigenschaft Körpergewicht, Größe und Blutviskosität sind. Durch die Berücksichtigung der Kombination zweier oder dreier körperlicher Eigenschaften wird eine noch genauere Einstellung und/oder Begrenzung der Blutpumpenförderrate ermöglicht. Eine erste Gruppe an körperlichen Eigenschaften kann dabei Körperoberfläche, Körpergewicht und Größe des Patienten sein. Eine weitere Gruppe an körperlichen Eigenschaften kann Körpergewicht, Größe und Gefäßzugang sein. Ebenfalls eine weitere Gruppe an körperlichen Eigenschaften können Körpergewicht, Größe und Blutviskosität sein. Grundsätzlich ist auch vorstellbar, sämtliche bislang genannten körperlichen Eigenschaften in Kombination und Zusammenhang auszuwerten. Wie bereits vorstehend ausgeführt, erlauben es die Eigenschaften Gefäßzugang und Blutviskosität vorgegebene fluiddynamische Randbedingungen bei der Bestimmung und Begrenzung der Blutpumpenförderrate mit einzubeziehen. Die übrigen körperlichen Eigenschaften wie Körperoberfläche, Körpergewicht, Größe des Patienten werden insbesondere herangezogen, um die zulässige Maximalmenge möglicherweise an infundierter Luft pro Zeiteinheit auf keinen Fall zu überschreiten, aber zugleich die maximal mögliche Blutpumpenförderrate zu bestimmen, um den Patienten vor einer unnötig langen und deshalb für den Patienten belastenden Dialysebehandlung zu bewahren.

Besonders vorteilhaft ist es, wenn die Bestimmung der körperlichen Eigenschaften und/oder die Einstellung und/oder Begrenzung der Blutpumpenförderrate automatisch mittels der Blutbehandlungsvorrichtung erfolgt. Hierdurch ergibt sich der Vorteil, daß der Bediener keine Körperdaten eingeben muß, was die Bequemlichkeit erhöht und die Gefahr von Fehleingaben verhindert.

Außerdem kann vorgesehen sein, daß das Erfassungsmittel ein Erfassungsmittel zur Erfassung der Körpergröße ist, wobei das Erfassungsmittel zur Erfassung der Körpergröße vorzugsweise eine Kamera, eine Messlatte mit entsprechenden Sensoren wie z. B. wenigstens ein Potentiometer, wenigstens einem Dehnungsmessstreifen, wenigstens einem optischen Sensor und/oder wenigstens einem magnetischen Sensor ist und/oder umfaßt.

Ferner ist denkbar, daß das Erfassungsmittel ein Anschlussmittel umfaßt, mittels dessen die Blutbehandlungsvorrichtung vorzugsweise mit einem Erfassungsmittel verbindbar ist und wobei mittels dieses Erfassungsmittels eine körperliche Eigenschaft eines mit der Blutbehandlungsvorrichtung zu behandelnden Patienten erfaßbar ist. Denkbar ist, daß also sonstige Vorrichtungen vorgesehen sind, die mit der Blutbehandlungsvorrichtung interagieren. Beispielsweise kann eine Patientenlagervorrichtung mit entsprechenden Mitteln wie einer Waage, Sensoren zur Größenbestimmung, beispielsweise in den Sessel integrierte Drucksensoren, vorgesehen sein, um Körpergröße, Körpergewicht und mittelbar und/oder unmittelbar die Körperoberfläche des Patienten bestimmen zu können.

Des Weiteren kann vorgesehen sein, daß das Erfassungsmittel ein Erfassungsmittel ist, mittels dessen die Blutviskosität erfaßbar ist. Hierzu können beispielsweise die mittels des arteriellen Drucksensors gemessenen Druckwerte herangezogen werden. Diese Werte liefern einen Anhaltspunkt für die Viskosität des Blutes, so daß mittelbar die Viskosität des Blutes errechenbar ist. So kann beispielsweise in einem Fall, in dem der arterielle Druck zu niedrig ist, entsprechend die Blutpumpenförderrate über das zuvor aus dem Körpergewicht und der Körpergröße bestimmte Maß zusätzlich begrenzt werden. Denkbar ist aber auch, die Viskosität des Blutes anhand des Drehmoments einer Zentrifugalpumpe, die als Blutpumpe dient, zu bestimmen.

Es ist ebenfalls denkbar, daß Daten, die direkt oder indirekt die Blutpumpenrate bestimmen, wie beispielsweise das Körpergewicht, die Körpergröße, die Körperoberfläche und/oder der Gefäßzugang, in einer Datenbank hinterlegt sind, auf die die Blutbehandlungsvorrichtung - beispielsweise über ein Netzwerk - Zugriff hat. Eine derartige Datenbank kann beispielsweise in einem Zentralrechner vorgehalten werden.

Weitere Einzelheiten und Vorteile der Erfindung sollen nun anhand eines nachstehend beschriebenen Ausführungsbeispiels der Erfindung erläutert werden.

Bei einer erfindungsgemäßen Blutbehandlungsvorrichtung, die als Dialysemaschine ausgeführt ist, sind demnach integrierte Mittel zur Bestimmung der Körpergröße und des Körpergewichts vorgesehen. Hierdurch ist die Dialysemaschine in der Lage, mit Hilfe dieser gemessenen Daten die Körperoberfläche des zu behandelnden Patienten zu bestimmen und entsprechend die Förderrate der Blutpumpe automatisch zu begrenzen. Derart ausgestaltet muß der Bediener keine Körperdaten eingeben, was die Bequemlichkeit der Bedienung der Dialysemaschine erhöht. Zugleich wird die Gefahr von Fehleingaben verhindert. Als Mittel zur Bestimmung der Körpergröße können hierbei Kameras, Messlatten mit entsprechenden Sensoren wie Potentiometern, Dehnungsmessstreifen, optische Sensoren, magnetische Sensoren, etc. oder sonstige Vorrichtungen vorgesehen sein, die mit der Dialysemaschine interagieren. Vorzugsweise ist dabei zugleich vorgesehen, daß die Patientenlagervorrichtung mit entsprechenden Mitteln ausgestattet ist, wie beispielsweise einer Waage, Sensoren zur Größenbestimmung, etwa in den Sessel integrierte Drucksensoren.

Zugleich ist die Blutbehandlungsvorrichtung in der Lage, die Blutpumpenförderrate auch anhand anderer Parameter zu begrenzen. Die Blutviskosität steht primär zwar nicht in direktem Zusammenhang mit der infundierten Luftmenge. Allerdings ist zu beachten, daß je dicker das Patientenblut ist, es um so weniger gut im extrakorporalen Blutkreislauf der Blutbehandlungsvorrichtung strömen kann, wodurch letztlich indirekt die maximale Blutpumpenförderrate und damit die möglicherweise infundierte Luftmenge beeinflust wird. Dabei liefert der arterielle Drucksensor einen Anhaltspunkt für die Viskosität des Blutes. Ist der arterielle Druck zu niedrig, so wird entsprechend die Blutpumpenförderrate der Dialysemaschine über das zuvor aus dem Körpergewicht und der Körpergröße bestimmte Maß zusätzlich begrenzt. Denkbar ist auch zusätzlich oder alternativ die Viskosität des Blutes anhand des Drehmomentes der Zentrifugalpumpe, die als Blutpumpe der Dialysemaschine dienen kann, festzustellen und diese Größe in die Bestimmung und Begrenzung der Blutpumpenförderrate mit eingehen zu lassen.

Zugleich wird bei der erfindungsgemäßen Dialysemaschine die Art des Gefäßzugangs des extrakorporalen Blutkreislaufs bei der Bestimmung und Begrenzung der Blutpumpenförderrate mit einbezogen. Dem liegt zugrunde, daß auch die Art des Gefäßzugangs des extrakorporalen Blutkreislaufs begrenzend auf die Förderrate der Blutpumpe wirkt. So ermöglicht beispielsweise ein einfacher Katheterzugang nur kleinere Blutförderraten als ein Fistelzugang oder der Zugang über ein künstliches Gefäßstück. Auch die Art des Gefäßzugangs kann der Maschine durch Bedienereingabe oder Patientenkarte bekannt gemacht werden.

Dem liegt insbesondere die Erkenntnis zugrunde, daß für die Höhe der Begrenzung der Förderrate der Blutpumpe der Dialysemaschine nicht nur allein entscheidend das Gewicht des Patienten ist, sondern beispielsweise auch die Körperoberfläche des Patienten. Die Körperoberfläche kann dabei beispielsweise aus der Berechnungsformel

$$Körperoberfläche(m²) = \sqrt{\frac{Größe(cm) * Gewicht(kg)}{3600}}$$

(Quelle: Mosteller RD. Simplified Calculation of body surface area. NEJM 1987; 317:1098.) errechnet werden.

Anhand der Körperoberfläche, des Gewichts und der Größe des Patienten kann sodann bereits nur anhand dieser Parameter, grundsätzlich aber auch unter Heranziehung weiterer Parameter die einzustellende maximale Blutpumpenrate ermittelt werden. Eine Anpassung kann sodann dadurch erfolgen, daß die bislang maximal mögliche Blutpumpenförderrate von 230 ml/min für Patienten mit einem Gewicht von 20 kg Körpergewicht entsprechend nach oben oder unten angepasst wird. Beispielsweise ergeben sich somit für ein Körpergewicht von 20 kg bis < 30 kg eine maximale Blutpumpenförderrate von 310 ml/min, von 30 kg bis < 40 kg eine Blutpumpenförderrate von 470 ml/min und bei einem Körpergewicht von ≥ 40 kg eine Blutpumpenförderrate von 600 ml/min.

Denkbar ist ferner, in Abhängigkeit des ermittelten bzw. eingegebenen Gewichts die Anzeige einer Gewichtsklasse durch die Dialysemaschine vorzunehmen. Denkbar ist, dabei drei Gewichtsklassen zu berücksichtigen. Hierbei kann eine erste Gewichtsklasse der Bereich von 10 kg - 20 kg, eine zweite Gewichtsklasse der Bereich von > 20 kg - 30 kg und eine dritte Gewichtsklasse von > 30 kg vorgesehen sein.

**Patentansprüche**

1. Blutbehandlungsvorrichtung, insbesondere Dialysemaschine, mit wenigstens einem Einstellmittel, mittels dessen eine Blutpumpenförderrate einstellbar ist, wenigstens einem Erfassungsmittel zur Erfassung von wenigstens einer körperlichen Eigenschaft, wobei die körperliche Eigenschaft eine körperliche Eigenschaft eines mit der Blutbehandlungsvorrichtung zu behandelnden Patienten ist und wobei die Blutbehandlungsvorrichtung derart ausgebildet ist, daß die Art des Gefäßzugangs über eine Bedienereingabe oder eine Patientenkarte in die Blutbehandlungsvorrichtung eingebbar ist und daß mittels des Einstellmittels anhand der erfaßten wenigstens einen körperlichen Eigenschaft und der Art des Gefäßzugangs die Blutpumpenförderrate einstellbar und/oder begrenzbar ist.

2. Blutbehandlungsvorrichtung nach Anspruch 1, **dadurch gekennzeichnet, daß** wenigstens zwei oder drei körperliche Eigenschaften erfassbar sind, anhand welcher die Blutpumpenförderrate einstellbar und/oder begrenzbar ist, wobei eine erste, zweite und eine dritte körperliche Eigenschaft Körperoberfläche, Körpergewicht und Größe sind oder wobei eine erste und eine zweite körperliche Eigenschaft Körpergewicht und Größe sind oder wobei eine erste, zweite und eine dritte körperliche Eigenschaft Körpergewicht, Größe und Blutviskosität sind.

3. Blutbehandlungsvorrichtung nach Anspruch 1, **dadurch gekennzeichnet, daß** eine der körperlichen Eigenschaften die Körperoberfläche, das Körpergewicht, die Körpergröße und/oder die Blutviskosität ist.

4. Blutbehandlungsvorrichtung nach einem der vorhergehenden Ansprüche, **dadurch gekennzeichnet, daß** die Bestimmung der körperlichen Eigenschaften und/oder die Einstellung und/oder Begrenzung der Blutpumpenförderrate automatisch mittels der Blutbehandlungsvorrichtung erfolgt.

5. Blutbehandlungsvorrichtung nach einem der vorhergehenden Ansprüche, **dadurch gekennzeichnet, daß** das Erfassungsmittel ein Erfassungsmittel zur Erfassung der Körpergröße ist, wobei das Erfassungsmittel zur Erfassung der Körpergröße vorzugsweise eine Kamera, eine Meßlatte mit entsprechenden Sensoren wie z. B. wenigstens einem Potentiometer, wenigstens einem Dehnungsmessstreifen, wenigstens einem optischen Sensor und/oder wenigstens einem magnetischen Sensor ist und/oder umfaßt.

6. Blutbehandlungsvorrichtung nach einem der vorhergehenden Ansprüche, **dadurch gekennzeichnet, daß** das Erfassungsmittel ein Erfassungsmittel zur Erfassung des Körpergewichts ist, wobei das Erfassungsmittel zur Erfassung des Körpergewichts vorzugsweise ein Drucksensor ist.

7. Blutbehandlungsvorrichtung nach einem der vorhergehenden Ansprüche, **dadurch gekennzeichnet, daß** das Erfassungsmittel ein Erfassungsmittel ist, mittels dessen die Blutviskosität erfaßbar ist.

8. Blutbehandlungsvorrichtung nach einem der vorhergehenden Ansprüche, **dadurch gekennzeichnet, daß** das Erfassungsmittel ein Anschlussmittel umfaßt, mittels dessen die Blutbehandlungsvorrichtung mit einem Erfassungsmittel verbindbar ist, und wobei mittels dieses Erfassungsmittels eine körperliche Eigenschaft eines mit der Blutbehandlungsvorrichtung zu behandelnden Patienten erfaßbar ist.

**Claims**

1. A blood treatment device, in particular a dialysis machine, comprising at least one adjusting means by means of which a blood pump delivery rate can be adjusted, at least one detection means for detecting at least one physical attribute, wherein the physical attribute is a physical attribute of a patient to be treated with the blood treatment device and wherein the blood treatment device is formed such that the type of vascular access can be entered into the blood treatment device via an operator input or a patient card and that by means of the adjusting means the blood pump delivery rate can be adjusted and/or limited with reference to the detected at least one physical attribute.

2. The blood treatment device according to claim 1, **characterized in that** at least two or three physical attributes can be detected, by means of which the blood pump delivery rate can be adjusted and/or limited, wherein a first, second and third physical attribute are the body surface area, body weight and height or wherein a first and a second physical attribute are body weight and height or wherein a first, second and third physical attribute are body weight, height and blood viscosity.

3. The blood treatment device according to claim 1, **characterized in that** one of the physical attributes is the body surface area, the body weight, the body height and/or the blood viscosity.

4. The blood treatment device according to any of the preceding claims, **characterized in that** the determination of the physical attributes and/or the adjustment and/or limitation of the blood pump delivery rate is effected automatically by means of the blood treatment device.

5. The blood treatment device according to any of the preceding claims, **characterized in that** the detection means is a detection means for detecting the body height, wherein the detection means for detecting the body height preferably is and/or comprises a camera, a gauge stick with corresponding sensors, such as at least one potentiometer, at least one strain gauge, at least one optical sensor and/or at least one magnetic sensor.

6. The blood treatment device according to any of the preceding claims, **characterized in that** the detection means is a detection means for detecting the body weight, wherein the detection means for detecting the body weight preferably is a pressure sensor.

7. The blood treatment device according to any of the preceding claims, **characterized in that** the detection means is a detection means by means of which the blood viscosity can be detected.

8. The blood treatment device according to any of the preceding claims, **characterized in that** the detection means comprises a connecting means by means of which the blood treatment device can be connected with a detection means, and wherein by means of this detection means a physical attribute of a patient to be treated with the blood treatment device can be detected.

**Revendications**

1. Dispositif de traitement du sang, en particulier machine de dialyse, comportant au moins un moyen de réglage, au moyen duquel le débit de la pompe sanguine peut être réglé, au moins un moyen de détection pour la détection d'au moins un attribut physique, l'attribut physique étant un attribut physique d'un patient à traiter avec le dispositif de traitement du sang et le dispositif de traitement du sang étant formé de telle sorte que le type d'accès vasculaire peut être entré dans le dispositif de traitement du sang par une entrée de l'opérateur ou une carte de patient et que le débit de la pompe sanguine peut être réglé et/ou limité au moyen du moyen de réglage sur la base d'au moins un attribut physique détecté et du type d'accès vasculaire.

2. Dispositif de traitement du sang selon la revendication 1, **caractérisé en ce qu'**au moins deux ou trois attributs physiques peuvent être détectés, sur la base desquels le débit de la pompe sanguine peut être réglé et/ou limité, dans lequel un premier, deuxième et troisième attribut physique sont la surface corporelle, le poids corporel et la taille ou dans lequel un premier et un deuxième attribut physique sont le poids corporel et la taille ou dans lequel un premier, deuxième et troisième attribut physique sont le poids corporel, la taille et la viscosité du sang.

3. Dispositif de traitement du sang selon la revendication 1, **caractérisé en ce que** l'un des attributs physiques est la surface corporelle, le poids corporel, la taille et/ou la viscosité du sang.

4. Dispositif de traitement du sang selon l'une quelconque des revendications précédentes, **caractérisé en ce que** la détermination des attributs physiques et/ou le réglage et/ou la limitation du débit de la pompe sanguine est effectué(e) automatiquement au moyen du dispositif de traitement du sang.

5. Dispositif de traitement du sang selon l'une quelconque des revendications précédentes, **caractérisé en ce que** le moyen de détection est un moyen de détection pour la détection de la taille, dans lequel le moyen de détection pour la détection de la taille de préférence est et/ou comporte une caméra, un étalon avec des capteurs correspondants, comme par exemple au moins un potentiomètre, au moins une jauge de contrainte, au moins un capteur optique et/ou au moins un capteur magnétique.

6. Dispositif de traitement du sang selon l'une quelconque des revendications précédentes, **caractérisé en ce que** le moyen de détection est un moyen de détection pour la détection du poids corporel, dans lequel le moyen de détection pour la détection du poids corporel de préférence est un capteur de pression.

7. Dispositif de traitement du sang selon l'une quelconque des revendications précédentes, **caractérisé en ce que** le moyen de détection est un moyen de détection au moyen duquel la viscosité du sang peut être détectée.

8. Dispositif de traitement du sang selon l'une quelconque des revendications précédentes, **caractérisé en ce que** le moyen de détection comporte un moyen de connexion au moyen duquel le dispositif de traitement du sang peut être connecté à un moyen de détection, et dans lequel au moyen de ce moyen de détection un attribut physique d'un patient à traiter avec le dispositif de traitement du sang peut être détecté.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0066197 A1
- US 2007215545 A1
- US 20110017667 A1

**In der Beschreibung aufgeführte Nicht-Patentliteratur**

- **MOSTELLER RD.** Simplified Calculation of body surface area. *NEJM,* 1987, vol. 317, 1098